# EUROPÄISCHE PATENTSCHRIFT

(11) **EP 1 429 994 B1**
(45) Veröffentlichungstag und Bekanntmachung des Hinweises auf die Patenterteilung: **18.04.2012**
(21) Anmeldenummer: 02799359.1
(22) Anmeldetag: 20.08.2002
(51) Int. Cl.: C01B 15/037

(54) **Verwendung von stabilisiertem Wasserstoffperoxid**
Use of stabilized hydrogen peroxide
Utilisation de peroxide d'hydrogène stabilisé

(30) Priorität: 21.09.2001 DE 10146594
(43) Veröffentlichungstag der Anmeldung: 23.06.2004
(73) Patentinhaber: Solvay Chemicals GmbH, 30173 Hannover (DE)
(72) Erfinder: DÖTSCH, Werner, 53545 Linz a. Rhein (DE); WOOST, Otmar, 06406 Bernburg (DE)
(74) Vertreter: Vande Gucht, Anne
(86) Internationale Anmeldenummer: PCT/EP2002/009284
(87) Internationale Veröffentlichungsnummer: WO 2003/027008

(56) Entgegenhaltungen:
- EP-A- 0 635 273
- EP-A- 0 906 763
- FR-A- 2 751 634
- US-A- 3 383 174
- US-A- 3 903 244
- PATENT ABSTRACTS OF JAPAN vol. 016, no. 020 (C-0902), 20. Januar 1992 (1992-01-20) & JP 03 237007 A (MITSUBISHI GAS CHEM CO INC), 22. Oktober 1991 (1991-10-22)
- "Amino-tris-methylenphosphonsäure (ATMP) und deren Salze" ZSM WEBSITE, [Online] XP002226219 Gefunden im Internet: <URL:http://www.zsm.de/german/phos/atmp.ht m> [gefunden am 2003-01-03]

## Beschreibung

Die Erfindung betrifft die Verwendung von stabilisiertem Wasserstoffperoxid in der chemischen Sterilisation von Packmaterialien, wie in dem Ansprüchen definiert.

Durch die chemische Sterilisation von Packmaterialien wird es heute ermöglicht, Lebensmittel wie z. B. Milch, Joghurt, oder Fruchtsäfte dem Endverbraucher in einer einfachen und benutzerfreundlichen Verpackung zur Verfügung zu stellen, ohne das jeweilige Lebensmittel selbst in irgendeiner Form zu behandeln oder zu beeinträchtigen.

Die hohe Akzeptanz der oben aufgeführten, benutzerfreundlichen Verpackung führt dazu, daß die Abfüllkapazität der Füllmaschinen ständig erhöht wird, was gleichzeitig oft mit einer Verkürzung der Füllzyklen einher geht.

Bei der chemischen Sterilisation von Packmaterialien sind aus lebensmittelrechtlichen Gründen die in Frage kommenden Chemikalien beschränkt. Zugelassen sind nur solche Chemikalien oder Mischungen, die alleine oder - im Falle von Mischungen- deren Einzelkomponenten lebensmittelrechtlich zugelassen sind.

Es hat sich in der Vergangenheit gezeigt, daß Wasserstoffperoxid aufgrund seines hohen Oxidationsvermögens ein sehr wirkungsvolles keimtötendes Medium ist und daher heute bei fast allen aseptisch arbeitenden Verpackungsanlagen in der milchverarbeitenden Industrie sowie in der Saftherstellung usw. seit Jahren mit Erfolg eingesetzt wird.

Gegenüber anderen keimtötenden Substanzen oder vergleichbaren Oxidationsmitteln weist Wasserstoffperoxid den großen Vorteil auf, auf dem Packmaterial produktbedingt und prozessbedingt außer Wasser keine Rückstände zu hinterlassen, sieht man von den geringfügigen Stabilisatorspuren ab.

In der chemischen Sterilisation von Packmaterialien haben sich heute im wesentlichen zwei Verfahren auf dem Markt etabliert, das Tauchbadverfahren sowie das Sprühverfahren. In beiden Verfahren wird heute bei erhöhten Temperaturen Wasserstoffperoxid als keimtötende Chemikalie eingesetzt, wobei die Anforderungen an die stoffspezifischen Eigenschaften des Wasserstoffperoxids vom jeweiligen Verfahren abhängen.

So sollte z. B. beim Sprühverfahren das eingesetzte Wasserstoffperoxid aus prozesstechnischen Gründen nur wenig "Inertmaterialien", die weitestgehend von den verwendeten Stabilisatoren herrühren, aufweisen: beim Sprühverfahren führen die "Inertmaterialien" zu Verkrustierungen im Verdampfer- bzw. Sprühteil, wodurch Reinigungsarbeiten anfallen und letztendlich die Füllkapazität verringert wird.

Im Tauchbadverfahren findet der Keimtötungsprozess in einem mit Wasserstoffperoxid gefüllten Bad statt. Das Packmaterial wird hierzu durch ein temperiertes Bad geleitet und im späteren Prozessverlauf mechanisch von anhaftenden Wasserstoffperoxidresten getrennt. Prozessbedingt muß daher das verwendete Wasserstoffperoxid höher stabilisiert sein als das bei dem oben aufgeführten Sprühverfahren eingesetzte Produkt.

Um die Standzeiten des eingesetzten Wasserstoffperoxides zu verlängern, wird das Wasserstoffperoxid mit lebensmitteltauglichen Stabilisatoren versetzt. Es ist z. B. bekannt, Pyrophosphate/Phosphorsäure in Kombination mit Stannaten zur Stabilisierung zu verwenden.

EP0635273 A1 offenbart, dass Packmaterialien mit stabilisiertem Wasserstoffperoxid sterilisiert werden können, wobei Wasserstoffperoxid als Tauchbadflüssigkeit oder als Aufsprühungsflüssigkeit verwendet wird.

Als Wasserstoffperoxid als Aufsprühungsflüssigkeit verwendet wird, sollte Wasserstoffperoxid mit weniger als 50 ppm Phosphonsäure stabilisiert sein.

Die oben beschriebenen Abfüllkapazitätserhöhungen gehen, bei nicht wesentlich geänderten Tauchbadgeometrien im wesentlichen mit einer Reduzierung der Verweilzeit des Packmaterials im Tauchbad einher. Um dennoch die Sterilisationswirkung aufrecht zu erhalten, muß die Arbeitstemperatur im Tauchbad erhöht werden.

Die Aufgabe der Erfindung besteht nun darin, Wasserstoffperoxid so zu modifizieren, daß seine Verwendung in schnellaufenden aseptischen Verpackungsanlagen bei höheren Temperaturen als bisher möglich ist ohne die Abfüllaufzeiten der Verpackungsanlage zu verkürzen. Als Meßgröße hierzu dient der Stabilitätsvergleich zu den z. Zt. für die "langsamer" laufenden Maschinen verwendeten Wasserstoffperoxidspezialitäten.

Überraschenderweise wurde gefunden, daß durch Zusatz von geringen Mengen an lebensmittelrechtlich zugelassenen Phosphonsäuren, vorzugsweise Aminotrismethylenphosphonsäure Wasserstoffperoxid so effizient und wirkungsvoll stabilisiert werden kann, daß auch bei Temperaturen von 85 °C gegenüber den heute in der Tauchbadtechnologie verwendeten Wasserstoffperoxidspezialtypen ein wesentlich geringerer Konzentrationsabbau beobachtet wird.

Diese Verbesserung im Stabilitätsverhalten ist nicht nur auf Wasserstoffperoxid beschränkt, welches noch nicht in den Prozess eingesetzt wurde und demnach noch keine Kontaminationen aus dem Verpackungsmaterial aufgenommen hat. Auch Wasserstoffperoxid, das prozessbedingt eine Anreicherung von Packmaterialresten aufzeigt, was zu einer heterogenen Zersetzung führt, verhält sich auch bei höheren Temperaturen gegenüber Standardwasserstoffperoxidqualitäten wesentlich stabiler.

Die zur Stabilisierung von Wasserstoffperoxid notwendige Stabilisatormenge beträgt 200 bis 500 ppm Aminotrismethylenphosphonsäure in Form einer 50%igen wäßrigen Lösung bezogen auf 1 1 Wasserstoffperoxid.

Ein weiter überraschender Vorteil des mit Aminotrismethylenphosphonsäure stabilisierten Wasserstoffperoxids ist darin zu sehen, daß bei der nach dem Tauchbadprozess erfolgenden Entfernung von Restwasserstoffperoxid auch im Dauerprozess keine harten Rückstände auf den Abstreiferwalzen aufgebaut werden.

Ein weiterer Vorteil stellt die Herstellung des mit Aminotrismethylenphosphonsäure stabilisierten Wasserstoffperoxids dar. In ein Wasserstoffperoxiddestillat wird die notwendige Stabilisatormenge eingemischt; ein Zusatz von weiteren Stabilisatoren kann entfallen.

Die nachfolgenden Beispiele sollen die Erfindung erläutern ohne sie einzuschränken.
1. In handelsübliches Wasserstoffperoxid der Type D0032 von der Fa. Solvay Interox, werden 250, 500 und 1000 µl einer wäßrigen 50%igen Lösung Aminotrismethylenphosphonsäure, Handelsname Cublen AP1 (Hersteller ZSCIMMER & SCHWARZ, MOHSDORF GmbH & Co KG, Mohsdorf) gelöst. Die Stabilitätswerte dieser Mischungen sind in Tabelle 1 wiedergegeben.
2. In handelsübliches Wasserstoffperoxid der Type D0035, welches seitens der Gerätehersteller für die Tauchbadtechnologie freigegeben ist, werden analog zu Beispiel 1 je 250, 500 und 1000 µl/l H₂O₂ einer wäßrigen 50%igen Lösung Aminotrismethylenphosphonsäure, Handelsname Cublen AP1 (Hersteller ZSCIMMER & SCHWARZ, MOHSDORF GmbH & Co KG, Mohsdorf) gelöst. Die Stabilitätswerte dieser Mischungen sind in Tabelle 2 wiedergegeben.
3. Zur Messung der Stabilität werden während der Bestimmung den Proben aus den oben aufgeführten Beispielen eine definierte Anzahl an Packmaterialschnipsel mit einer Oberfläche von 55 - 60 mm²/Schnipsel zugesetzt. Zur Stabilitätsmessung werden etwa 50 ml der Probenlösung, mit bekannter Konzentration (Wₐ) bei einer Lagertemperatur von 70 °C bzw. 85 °C über einen Zeitraum von 960 min. in einem Glaskolben gelagert. Anschließend wird nach der notwendigen Volumenkorrektur, hervorgerufen durch verdampftes Wasser der Wasserstoffperoxidgehalt erneut durch hierfür übliche Methoden bestimmt (Wₑ). Der Stabilverlust errechnet sich über: (Wₐ - Wₑ)/Wₐ x 100.

Wₐ = Anfangskonzentration des eingesetzten Wasserstoffperoxids.
Wₑ = Endkonzentration des Wasserstoffperoxids nach 16-h Test bei der jeweiligen Versuchstemperatur.

**Tabelle 1:**

| | | | **H₂O₂ Typ D0032** | **H₂O₂ Typ D0035** |
|---|---|---|---|---|
| Stabilisatorzugabe (µl/l H₂O₂) | | | Stabilverlust (%) | Stabilverlust (%) |
| 0 | | | 38 | 23 |
| 250 | | | 12 | 15 |
| 500 | | | 5 | 11 |
| 1000 | | | 6 | 3 |
| Stabilisator: | | Cublen AP1 (50%ige Lösung) | | |
| Testtemperatur: | | T = 85 °C | | |
| Testzeit: | | † = 960 min | | |
| Packmaterialschnipsel: | | n = 50 | | |

**Tabelle 2:**

| | | | **H₂O₂ Typ D0035** |
|---|---|---|---|
| Stabilisatorzugabe (µ/l H₂O₂) | | | Stabilverlust(%) |
| 0 | | | 6 |
| 250 | | | |
| 500 | | | 2 |
| 1000 | | | 1 |
| Stabilisator: | | Cublen AP1 (50%ige Lösung) | |
| Testtemperatur: | | T = 70 °C | |
| Testzeit: | | † = 960 min | |
| Packmaterialschnipsel: | | n = 50 | |

**Tabelle 3:**

| **Temperature (°C)** | **Anzahl der Schnipsel** | **Stabilverlust (%)** |
|---|---|---|
| 70 | 0 | 1 |
| | 25 | 1 |
| | 50 | 6 |
| 85 | 0 | 1 |
| | 25 | |
| | 50 | 23 |
| 96 | 0 | 1 |
| | 25 | |
| | 50 | 50 |
| Wasserstoffperoxidtyp D0035 | | |
| Testzeit: † = 960 min | | |

## Patentansprüche

1. Verwendung von stabilisiertem Wasserstoffperoxid, das mit 200 bis 500 ppm lebensmitteltauglichen Phosphonsäuren stabilisiert ist, zur chemischen Sterilisierung von Packmaterialien.

2. Verwendung nach Anspruch 1, **dadurch gekennzeichnet, dass** das Wasserstoffperoxid mit Aminotrismethylenphosphonsäure stabilisiert ist.

3. Verwendung von mit lebensmitteltauglichen Phosphonsäuren stabilisiertem Wasserstoffperoxid gemäß Anspruch 1 oder 2 als Tauchbadflüssigkeit zur chemischen Sterilisierung von Packmaterialien in schnellaufenden aseptischen Verpackungsanlagen.

4. Verwendung von stabilisiertem Wasserstoffperoxid als Tauchbadflüssigkeit nach Anspruch 3, **dadurch gekennzeichnet, dass** die Tauchbadflüssigkeit eine Temperatur von 80 bis 85°C hat.

5. Verfahren zur chemischen Sterilisierung von Packmaterialien, welches beinhaltet, dass das Packmaterial durch ein mit Wasserstoffperoxid gefülltes Bad geleitet wird, wobei das Wasserstoffperoxid mit 200 bis 500 ppm lebensmitteltauglichen Phosphonsäuren stabilisiert ist.

6. Verfahren nach Anspruch 5, **dadurch gekennzeichnet, dass** das Wasserstoffperoxid mit Aminotrismethylenphosphonsäure stabilisiert ist.

7. Verfahren nach Anspruch 5 oder 6, **dadurch gekennzeichnet, dass** die Tauchbadflüssigkeit eine Temperatur von 80 bis 85°C hat.

## Claims

1. Use of stabilized hydrogen peroxide stabilized with 200 to 500 ppm of food stuff-compatible phosphonic acids for the chemical sterilization of packaging materials.

2. Use according to Claim 1, **characterized in that** the hydrogen peroxide is stabilized with aminotrismethylenephosphonic acid.

3. Use of hydrogen peroxide stabilized with foodstuff-compatible phosphonic acids according to Claim 1 or 2 as dip bath liquid for the chemical sterilization of packaging materials in high-speed aseptic packaging plants.

4. Use of stabilized hydrogen peroxide as dip bath liquid according to Claim 3, **characterized in that** the dip bath liquid has a temperature of 80 to 85°C.

5. Process for the chemical stabilization of packaging materials which involves passing the packaging material through a bath filled with hydrogen peroxide, where the hydrogen peroxide is stabilized with 200 to 500 ppm of foodstuff-compatible phosphonic acids.

6. Process according to Claim 5, **characterized in that** the hydrogen peroxide is stabilized with aminotrismethylenephosphonic acid.

7. Process according to Claim 5 or 6, **characterized in that** the dip bath liquid has a temperature of 80 to 85°C.

## Revendications

1. Utilisation de peroxyde d'hydrogène stabilisé, qui est stabilisé avec 200 à 500 ppm d'acides phosphoniques aptes au contact alimentaire, pour la stérilisation chimique de matériaux d'emballage.

2. Utilisation selon la revendication 1, **caractérisée en ce que** le peroxyde d'hydrogène est stabilisé avec de l'acide aminotrisméthylènephosphonique.

3. Utilisation de peroxyde d'hydrogène stabilisé avec des acides phosphoniques aptes au contact alimentaire selon la revendication 1 ou 2 en tant que liquide de bain d'immersion pour la stérilisation chimique de matériaux d'emballage dans des unités d'emballage aseptiques rapides.

4. Utilisation de peroxyde d'hydrogène stabilisé en tant que liquide de bain d'immersion selon la revendication 3, **caractérisée en ce que** le liquide de bain d'immersion a une température de 80 à 85°C.

5. Procédé de stérilisation chimique de matériaux d'emballage, qui comprend le passage du matériau d'emballage dans un bain rempli avec du peroxyde d'hydrogène, le peroxyde d'hydrogène étant stabilisé avec 200 à 500 ppm d'acides phosphoniques aptes au contact alimentaire.

6. Procédé selon la revendication 5, **caractérisé en ce que** le peroxyde d'hydrogène est stabilisé avec de l'acide aminotrisméthylènephosphonique.

7. Procédé selon la revendication 5 ou 6, **caractérisé en ce que** le liquide du bain d'immersion a une température de 80 à 85°C.
